# EUROPEAN PATENT SPECIFICATION

(11) **EP 1 697 509 B1**
(45) Date of publication and mention of the grant of the patent: **20.04.2011**
(21) Application number: 04803042.3
(22) Date of filing: 20.12.2004
(51) Int. Cl.: C12N 9/48

(54) **PROCESSING OF PEPTIDES AND PROTEINS**
PROZESSIERUNG VON PEPTIDEN UND PROTEINEN
TRAITEMENT DE PEPTIDES ET DE PROTEINES

(30) Priority: 19.12.2003 DK 200301892
(43) Date of publication of application: 06.09.2006
(73) Proprietor: NOVO NORDISK A/S, 2880 Bagsværd (DK)
(72) Inventor: SIG NIELSEN NØRBY, Inga, 3460 Birkerød (DK); FOGH IVERSEN, Lars, 2840 Holte (DK)
(74) Representative: Winther, Kamilla
(86) International application number: PCT/DK2004/000891
(87) International publication number: WO 2005/059127

(56) References cited:
- WO-A-99/61617
- WO-A1-95/30685
- LOWTHER W T ET AL: "Structure and function of the methionine aminopeptidases" BIOCHIMICA ET BIOPHYSICA ACTA. PROTEIN STRUCTURE AND MOLECULAR ENZYMOLOGY, ELSEVIER, AMSTERDAM,, NL, vol. 1477, no. 1-2, 7 March 2000 (2000-03-07), pages 157-167, XP004278892 ISSN: 0167-4838
- CHIU CHEN-HSIANG ET AL: "Amino acid residues involved in the functional integrity of Escherichia coli methionine aminopeptidase" JOURNAL OF BACTERIOLOGY, vol. 181, no. 15, August 1999 (1999-08), pages 4686-4689, XP002320613 ISSN: 0021-9193
- WALKER K W ET AL: "Yeast methionine aminopeptidase I. Alteration of substrate specificity by site-directed mutagenesis." THE JOURNAL OF BIOLOGICAL CHEMISTRY. 7 MAY 1999, vol. 274, no. 19, 7 May 1999 (1999-05-07), pages 13403-13409, XP002320532 ISSN: 0021-9258 & WALKER K W ET AL: "Erratum: Yeast methionine aminopeptidase I. Alteration of substrate specificity by site-directed mutagenesis (Journal of Biological Chemistry (1999) 274 (13403-13409))" JOURNAL OF BIOLOGICAL CHEMISTRY 17 SEP 1999 UNITED STATES, vol. 274, no. 38, 17 September 1999 (1999-09-17), page 27338, ISSN: 0021-9258
- LOWTHER W T ET AL: "Escherichia coli methionine aminopeptidase: implications of crystallographic analyses of the native, mutant, and inhibited enzymes for the mechanism of catalysis." BIOCHEMISTRY. 15 JUN 1999, vol. 38, no. 24, 15 June 1999 (1999-06-15), pages 7678-7688, XP002320531 ISSN: 0006-2960
- LIAO Y-D ET AL: "REMOVAL OF N-TERMINAL METHIONINE FROM RECOMBINANT PROTEINS BY ENGINEERED E. COLI METHIONINE AMINOPEPTIDASE" PROTEIN SCIENCE, CAMBRIDGE UNIVERSITY PRESS, CAMBRIDGE, GB, vol. 13, no. 7, July 2004 (2004-07), pages 1802-1810, XP008043171 ISSN: 0961-8368
- LIU L-F ET AL: "Escherichia coli methionine aminopeptidase with Tyr168 to alanine substitution can improve the N-terminal processing of recombinant proteins with valine at the penultimate position" ANALYTICAL BIOCHEMISTRY, ACADEMIC PRESS, NEW YORK, NY, US, vol. 329, no. 2, 15 June 2004 (2004-06-15), pages 345-347, XP004509836 ISSN: 0003-2697
- PARRISH-NOVAK J. ET AL NATURE vol. 408, 02 November 2000, pages 57 - 63

## Description

### FIELD OF THE INVENTION

The invention relates to a method for processing initiator methionine containing proteins by the enzyme Methionine Aminopeptidase and mutants thereof to yield initiator methionine free peptides.

### BACKGROUND OF THE INVENTION

Production of peptides by recombinant techniques using either prokaryotic or eukaryotic expression systems inherently yields the peptide with a leading methionine amino acid. This amino acid may not be present in the native protein i.e. the variant of the peptide processed for translocation. Obtaining the peptide without the leading methionine thus requires a further processing step. In the present invention the step is performed by the enzyme Methionine Aminopeptidase, which selectively cleaves the initiator methionine from the peptide.

Methionine Aminopeptidases (Met-AP's) are known in the art as enzymes which cleave leading methionines, if the leading peptide sequence is of a certain predetermined character. Wild-type *Escherichia coli* Met-AP selectively cleaves after an initiator Met residue if the residue in the P1' position is Gly, Ala, Ser, Thr, Pro, Val or Cys.

In the present invention the methionine aminopeptidases are improved by introducing mutations in the substrate binding sites which results in methionine aminopeptidases which cleaves the methionine regardless of the leading peptide sequence (P1' position).

Walker et al, Journal of Biological Chemistry, 274(19), 13403-13409 (1999) concerns yeast MetAP I enzymes mutated on residues M329 and/or Q356 having altered substrate specificity.

WO95/30685 concerns a method for purifying His-tagged recombinant peptides and then removing the His-tag by treatment with aminopeptidase, glutamine cyclotransferase (Qcyclase) and pyroglutamine aminopeptidase (PGAP).

### SUMMARY OF THE INVENTION

The invention provides a method for removal of initiator methionine from a substrate protein and subsequent purification of the product wherein the removal of the initiator methionine is performed by use of an E. coli methionine aminopeptidase, wherein the residues in positions 168, 206, or 233 has been amended into a sequence different from Y168,and M206 and Q233, and wherein the method for separating the substrate protein starting with Met-Gln from the product protein, identical to the substrate but starting with Gln, comprises the step of applying Qcyclase to convert the N-terminal Gln of the product into pyroglutamine, followed by purification utilising the charge difference between the substrate and the product.

### DESCRIPTION OF THE DRAWINGS

Figure 1: Example of a *E*. *coli* Met-AP mutant expression construct layout. Purification indicates tag for purification purposes. Protease indicates protease cleavage site.
Figure 2: Expression in e.g. *E*. *coli* of (A) NT1-Enterokinase-Met-AP Y168A or (B) NT1-Enterokinase-Met-AP Y168G, M206N, Q233A as indicated.
Figure 3: hexa-His-Xa-Met-AP (M206A, Q233A) cleavage of Met-hIL-21
Figure 4: Maldi-tof es mass spectrum of purified hexa-His-Xa-Met-AP M206A, Q233A.
Figure 5: Purification chromotogram of the separation of the three compounds Met-IL-21, IL-21 and pyroglutamine IL-21.

### DEFINITIONS

P1 defines the first amino acid N-terminal to the recognition site for the enzyme. P1' denotes the amino acid adjacent to P1 towards the C-terminal. P1 is methionine.

Substrate specificity means selectivity towards the P1' position - which is the position just C-terminal to methionine. Wild-type *Escherichia coli* Met-AP exhibits the substrate specificity, that it selectively cleaves after an initiator Met residue if the residue in the P1' position is Gly, Ala, Ser, Thr, Pro, Val or Cys. Mutants showing an extended substrate specificity means that further amino acid can occupy the P1' position and still cleavage of the methionine is observed.

Variant means a sequence which has maintained the qualitative activity of the parent sequence, ie as methionine aminopeptidase, but wherein the sequence differs from the parent sequence by deletions, insertions, extension or substitution of one or more amino acids of the parent sequence. Variants in principle also include fragments of any length provided the activity is maintained.

Chemical derivatives of a specific protein means a derivative of the native protein which is not a variant, and which maintains the qualitative activity of the parent protein sequence. The chemical derivative includes derivatives such as PEG-groups.

The terms peptide and proteins are used interchangeable and is not meant as indications or limitations as to size or function of the sequences.

### DESCRIPTION OF THE INVENTION

The Met-Ap from *E*. *coli* has a substrate defining pocket (as part of active site) essentially, but probably not exclusively, defined by the amino acids Tyr 168, Met 206 and Gln 233. Mutating these positions extends the enzymes substrate specificity. The novel E.coli aminopeptidases described in the present invention extends the applicability of the Methionine aminopeptidases to be useful for removing the initiating methionine from almost any type of protein or peptide regardless of the amino acid sequence downstream of the methionine (P1' position). Hence, the initiator methionine can be removed from all initiator methionine containing peptides or proteins to produce initiator methionine-free peptides or proteins.

The *E*. *coli* Methionine aminopeptidase gene was cloned and mutant versions have been created using site directed mutagenesis.

The mutants were expressed in *E*. *coli* and the resulting enzymes were purified by conventional His-tag system. The enzyme can also be tagged by for example the FLAG-system or tagged and purified by other technologies as described in WO 03042249. Catalytic activity was monitored using initiator Met containing hIL-21 as a substrate.

In principle, the invention is generally applicable to any peptide. The invention is demonstrated as being useful for cleavage of the initiator methionine for peptides such as hIL-21. hIL-21 is a model system for P1' position being a Gln. IL-21 is described in WO00/53761 and is described as being effective in the treatment of cancer and viral infection among others. IL-20 is described in WO9927103. hGH refers to human Growth Hormone. Both are model systems for other aminoacids in P1' position.

In one aspect, the invention provides a method for removal of initiator methionine from a substrate protein and subsequent purification of the product wherein the removal of the initiator methionine is performed by use of an E. coli methionine aminopeptidase, wherein at least one of the residues in positions 168, 206, or 233 has been amended into a sequence different from Y168,and M206 and Q233, and wherein the method for separating the substrate protein starting with Met-Gln from the product protein, identical to the substrate but starting with Gln, comprises the step of applying Qcyclase to convert the N-terminal Gln of the product into pyroglutamine, followed by purification utilising the charge difference between the substrate and the product.

In one embodiment, the E. coli methionine aminopeptidase comprises an amendment of the amino acid in position 168.

In another embodiment, the E. coli methionine aminopeptidase comprises an amendment in position 206.

In another embodiment, the E. coli methionine aminopeptidase comprises an amendment in position 233.

In another embodiment, the E. coli methionine aminopeptidase comprises amendments in position 206 and 233.

In another embodiment, the E. coli methionine aminopeptidase comprises amendments in positions 168 and 206.

In another embodiment, the E. coli methionine aminopeptidase comprises amendments in positions 168 and 233.

In another embodiment, the E. coli methionine aminopeptidase comprises amendments in positions 168, 206, and 233.

In another embodiment, the E. coli methionine aminopeptidase comprises amendments comprises exchange of wildtype amino acid into Gly, Ala, Ser, Thr, Asn, or Asp.

In another embodiment, the E. coli methionine aminopeptidase comprises exchange of wildtype amino acid into Ala and/or Gly.

In another embodiment, the E. coli methionine aminopeptidase comprises exchanges of wildtype amino acid into Ala.

In another embodiment, position 168 of the E. coli methionine aminopeptidase is Ala.

In another embodiment, position 206 of the E. coli methionine aminopeptidase is Ala.

In another embodiment, position 233 of the E. coli methionine aminopeptidase is Ala.

In another embodiment, positions 206 and 233 of the E. coli methionine aminopeptidase are both Ala.

In another embodiment, the E. coli methionine aminopeptidase has the amino acid sequence shown in Seq. id. No. 3.

In another embodiment, the E. coli methionine aminopeptidase has the amino acid sequence shown in Seq. id. No. 5.

In another embodiment, the E. coli methionine aminopeptidase has the amino acid sequence shown in Seq. id. No. 7.

In another embodiment, the E. coli methionine aminopeptidase has the amino acid sequence shown in Seq. id. No 9.

In another embodiment, the method according to comprises purification on a cation exchange column.

Described herein is the methionine aminopeptidase enzyme having the following sequence (also described as seq. id. no. 1) wherein Xₐ, X_{b} and X_{c} are variable amino acids, and wherein Xₐ, X_{b} and X_{c} are not simultaneously Tyr, Met and Gln respectively. In an aspect of the invention one or more of Xₐ, X_{b} and X_{c} are exchanged from the wild type amino acid into Gly, Ala, Ser, Thr, Asn or Asp. In an aspect of the invention Xₐ, X_{b} and X_{c} are exchanged from the wild type amino acid into Gly or Ala; . In an aspect of the invention Xₐ, X_{b} and X_{c} are exchanged from the wild type amino acid into Ala. The present invention thus provides substitution Y168 to Ala (Y168A)(Seq. id no. 9) The corresponding DNA encoding the above is seq. id. no. 8;

The present invention thus provides substitution Met 206 to Ala (M206A)(Seq. id no. 3) which extends the enzymes substrate specificity to allow the following amino acids: Asn, Leu, Ile and Phe in the P1' postion.

The present invention also provides substituting Gln 233 to Ala (Q233A) (Seq. id. No.5) or both Met 206 and Gln 233 into Ala (M206A Q233A)(Seq. id no. 7): which further allow the P1' amino acids to be His, Gln and Trp.

In aspects of the invention postion 168 is amended into Gly (Y168G) or Ala (Y168A) or Asn (Y168N). Aspects of the invention are wherein amino acid 206 is an Ala (M206A) or a Gly (M206G) or Asn (M206N), and/or wherein amino acid 233 is an Ala (Q233A) or a Gly (Q233G) or Asn (Q233N). Aspects of the invention comprise the combination of two or three amendments according to the below, - wherein the wild-type combination of (Y168 M206 233Q) is not within the invention.

| **Position 168** | **Position 206** | **Position 233** |
|---|---|---|
| A/G/N/Y | A/G/N/M | A/G/N/Q |

Accordingly, aspects of the invention are wherein position 206 and position 233 are both Ala (M206A Q233A) or Gly or Asn, or combinations thereof: (M206G Q233A), (M206G Q233G), (M206A Q233G), (M206N Q233A), (M206N Q233N), (M206A Q233N). Aspects of the invention are wherein position 168 is amended according to the below:

| **Position 168** | **Position 206** | **Position 233** |
|---|---|---|
| A/G/N | A | Q |
| A/G/N | G | Q |
| A/G/N | N | Q |
| A/G/N | M | A |
| A/G/N | M | G |
| A/G/N | M | N |
| A/G/N | A | A |
| A/G/N | G | A |
| A/G/N | N | A |
| A/G/N | A | G |
| A/G/N | A | N |
| A/G/N | N | G |
| A/G/N | N | N |
| A/G/N | G | G |
| A/G/N | G | N |

Aspects of the invention are wherein at least one of the amended positions are amended into an Ala.

Aspects of the invention are the following mutants: (Y168G M206A), (Y168G M206A 233A), (Y168G M206N), (Y168G M206N 233A), (Y168A M206A 233A), (Y168A M206A), (Y168A M206N), (Y168A M206N 233A) and (M206A Q233A);

The invention thus provides a novel enzymes capable of cleaving a peptide containing an initiating methionine followed by a Asn, Leu, Ile, Phe, His, Gln or Trp in the P1' postion as well as the amino acids allowed by the wild type E.coli aminopeptidase. Wildtype E.coli methionine aminopeptidase allows the P1' to be any of the following amino acids: Gly, Ala, Ser, Thr, Pro, Val or Cys.

The mutant Methionine aminopeptidases are expressed in *E. coli,* but in principle the host cells could be of other prokaryotic origin or eukaryotic origin such as *Saccharomyces cerevisiae, Schizosaccharomyces pombe, Pichia pastoris* etc. or for example mammalian cells.

Removal of initiator methionine by methionine aminopeptidase may be performed *in vitro* following methionine aminopeptidase expression in, and purification from, prokaryotic or eukaryotic cells. This procedure is demonstrated below. Alternatively removal of initiator methionine may take place *in vivo* either in cells expressing a di-cistronic plasmid or in cells co-expressing plasmids carrying the methionine aminopeptidase and the substrate peptide or protein. *In vivo* initiator methionine processing may also be performed in cells where the genes encoding the methionine aminopeptidase and the peptide or protein to be processed have been integrated into the genome.

Experiments have been performed which provides a set of optimum conditions for the reaction: The optimal temperature for the reaction was determined to be between 15 and 24 degrees Celsius. Typically the reaction was hereafter performed at 18 degrees Celsius.

The concentration of ZnCl₂ was determined to be optimal at around 7.5µM and NaCl concentration was found optimal around 100mM and acceptable under 130mM.

After cleavage of the initiator methionine separation of the product from the starting material can be achieved by exploiting the different biophysical properties of the two peptides.

The peptide can be hIL-21, which after removal of the initial methionine contains a Gln in the N-terminal. Treatment with Qcyclase forms a pyroglutamine (pGlu). Due to cyclic amid formation the products net change is negative relative to the Methionine containing peptide. The difference in charge affects the eluation on a cation exchange column, due to Methionine containing peptide having a stronger binding to the cation resin. Further, in non-cyclised hIL-21 the N-terminal positioned Gln residue will have the ability to form a hydrogen bond between the side chain amide oxygen and the charged N-terminal backbone amine and thereby masking the charge at the N-terminus. Met-hIL-21 will not possess the ability for a similar charge masking and will therefore bind stronger to the cation exchange column than hIL-21.

### EXAMPLES

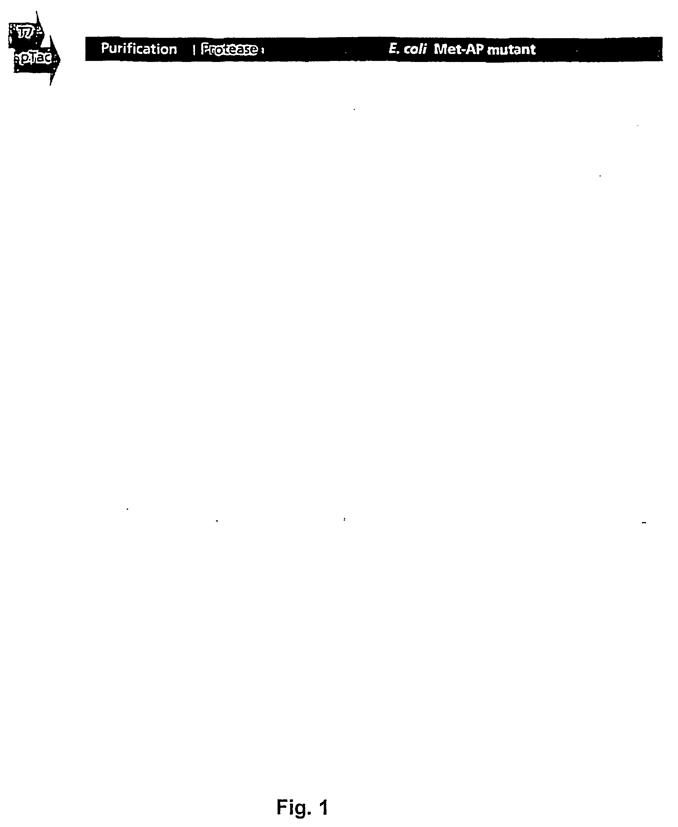

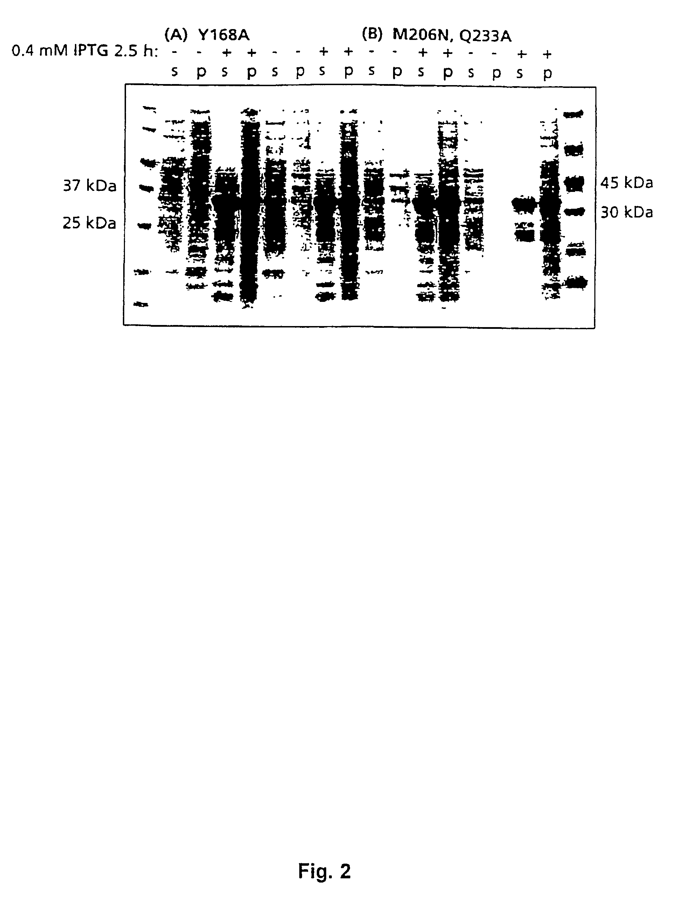

Various Met-AP expression constructs, as outlined in figure 1, have been created. NT1 (HHHNSWDHDINR) or hexa-His tag has been added to the various mutant forms of Met-AP for purification purposes. The purification tag may be removed using Factor Xa in some constructs or Enterokinase in others, or the purification tag may be left on the enzyme. mRNA expression was under the control of the T7 or the *tac* promoter. Constructs under the control of the T7 promoter were expressed in BL21(DE3) whereas constructs under the control of the *tac* promoter were expressed in BL21. Expression was induced by addition of IPTG to 0.4 mM to cultures (6 mL) grown to OD₆₀₀ 0.4 in LB-medium. Cells were harvested by centrifugation after 2.5 hours. Cell lysis was done by multiple freeze-thaw cycles and soluble or insoluble protein fractions were separated by centrifugation. Soluble or insoluble protein, before or after induction of expression, originating from equal amounts cells (measured by OD₆₀₀) were subjected to SDS-PAGE and subsequent colloidal blue staining (Fig. 2). Met-AP expression levels were estimated at ~250 mg/L after 2.5 h of induction in 6 mL cultures.

*E. coli* cells harvested from 1 L of culture expressing hexa-His-Met-AP M206A, Q233A were lysed using a cell disruptor, and the clarified lysate was applied on a Ni²⁺-NTA superflow column. Elution with an imidazole gradient released the Met-AP fusion protein at approximately 200 mM imidazole. The enzyme was further purified and buffer exchanged (into storage/cleavage buffer) using size exclusion chromatography. The enzyme was analysed using SDS-PAGE, MALDI-MS and N-terminus sequencing - verifying the molecular mass and identity of the enzyme.

According to the procedure above NT1-Enterokinase-Met-AP mutants were prepared. Expression was under the control of the tac promoter. Addition of IPTG to the cultures induced primarily soluble expression of the Met-AP enzymes. The following mutants were prepared according to the above: (Y168G M206A), (Y168G M206A 233A), (Y168G M206N), (Y168G M206N 233A), (Y168A M206A 233A), (Y168A M206A), (Y168A M206N) and (Y168A M206N 233A)

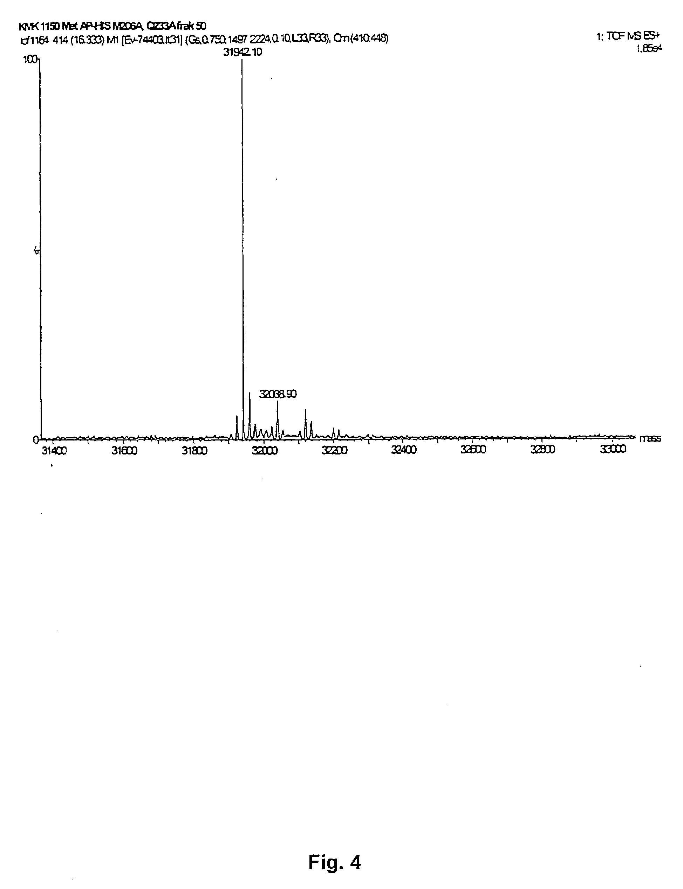

Hexa-His-Xa-MetAP Q233A was affinity purified using Ni²⁺-NTA superflow. Maldi-tof es mass spectrum of purified hexa-His-Xa-Met-AP M206A, Q233A shows that the correct enzymes were isolated. A mass of 32038.90 corresponds to Met-hexa-His-Xa-Met-AP M206A, Q233A and a mass of 31942.10 corresponds to hexa-His-Xa-Met-AP M206A, Q233A indicating that hexa-His-Xa-Met-AP M206A, Q233A was processed by WT Met-AP or hexa-His-Xa-Met-AP M206A, Q233A *in vivo.* The result is demonstrated in Figure 4.

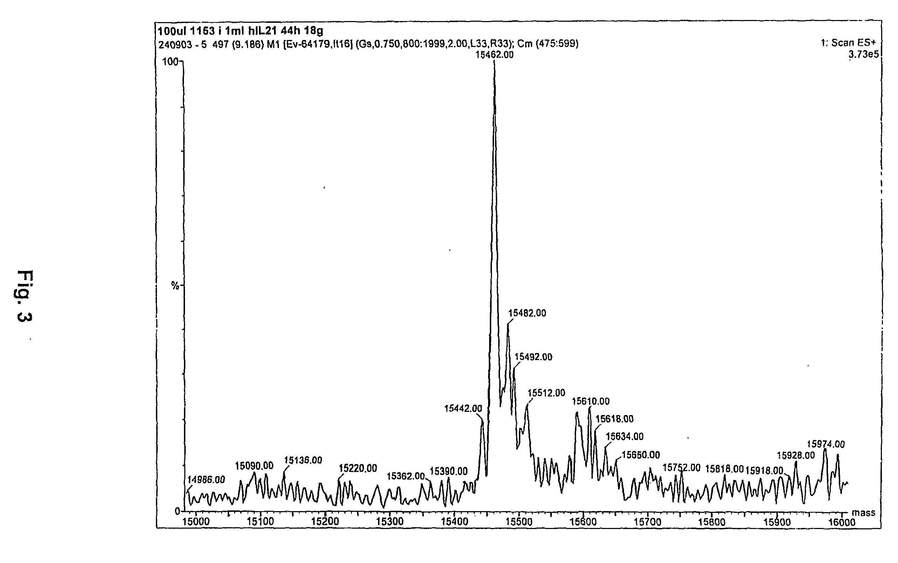

Addition of hexa-His-Xa-Met-AP M206A, Q233A to Met-hIL-21 at pH 7, 18°C generated ~65 % Met-free hIL-21. In 44 h. more than 90% cleavage of Met-hIL-21 could be observed (Figure 3).

Another mutant prepared by this method was hexa-His-Xa-Met-AP Q233A;

### Removal of initiator Met from Met-IL21 by Met-AP (M206A, Q233A).

Purified Met-AP (M206A, Q233A) was used to remove the initiator Methionine from partly or fully purified Met-IL21. The cleavage was performed in a reaction buffer typically consisting of the following components: 2-100 mM K₂SO₄, 2-500 mM NaCl, 1-100 µM ZnCl₂ and 2-30 mM Hepes buffer pH 6-8. The cleavage was assayed by MALDI-TOF spectroscopy. The time of reaction was 2-66 hours. Using these condition removal of Methionine from Met-IL21 below detection limits of Met-IL21 could be performed.

**Removal of initiator Met from Met-IL21 by Met-AP (M206A).**

Purified Met-AP (M206A) was used to remove the initiator Methionine from partly or fully purified Met-IL21. The cleavage was performed in a reaction buffer typically consisting of the following components: 2-100 mM K₂SO₄, 2-500 mM NaCl, 1-100 µM ZnCl₂ and 2-30 mM Hepes buffer pH 6-8. The cleavage was assayed by MALDI-TOF spectroscopy. The time of reaction was 2-66 hours. Using these condition removal of Methionine from Met-IL21 below detection limits of Met-IL21 could be performed.

### Influence of temperature on removal of initiator Met from Met-IL21 by Met-AP (M206A, Q233A).

Using the conditions and assay described in example 1 the temperature was varied between 4, 15, 24 and 30 degrees Celsius, respectively while the other parameters was fixed. The optimal temperature for the reaction was determined to be between 15 and 24 degrees Celsius. Typically the reaction was hereafter performed at 18 degrees Celsius.

### Influence of ZnCl₂ concentration on removal of initiator Met from Met-IL21 by Met-AP (M206A, Q233A).

Using the conditions and assay described in example 1 the ZnCl₂ concentration was varied between 7.5, 11 and 15 µM, respectively while the other parameters were fixed. The optimal ZnCl₂ concentration for the reaction was determined to be 7.5 µM. Typically, the reaction was hereafter performed at 7.5 µM ZnCl₂.

### Influence of NaCl concentration on removal of initiator Met from Met-IL21 by Met-AP (M206A, Q233A).

Using the conditions and assay described in example 1 the NaCl concentration was varied between 80, 130 and 180 mM, respectively while the other parameters were fixed. The maximum NaCl concentration tolerated for the reaction to run was determined to be 130 mM. Typically the reaction was hereafter performed at 100 mM NaCl.

### Influence of the addition of Q-cyclase on removal of initiator Met from Met-IL21 by Met-AP (M206A, Q233A) and the formation of pyro-glutamine.

Using the conditions as described in examples above the effect of adding Q-cyclase to the reaction mixture was determined. Again MALDI-TOF was used for assaying the removal of Methonine and subsequently conversion of glutamine in position 1 in IL21 into pyro-glutamine. It was found that the addition of Q-cyclase to the reaction mixture did not negatively influence the removal of initiator Methionine from Met-IL21 and further the Q-cyclase was fully efficient in converting converting glutamine in position 1 in IL21 into pyro-glutamine under the reaction conditions described in the examples above.

### Purification and separation of Met-IL21, IL21 and pyro-glutamine IL-21 using a Mono-S column.

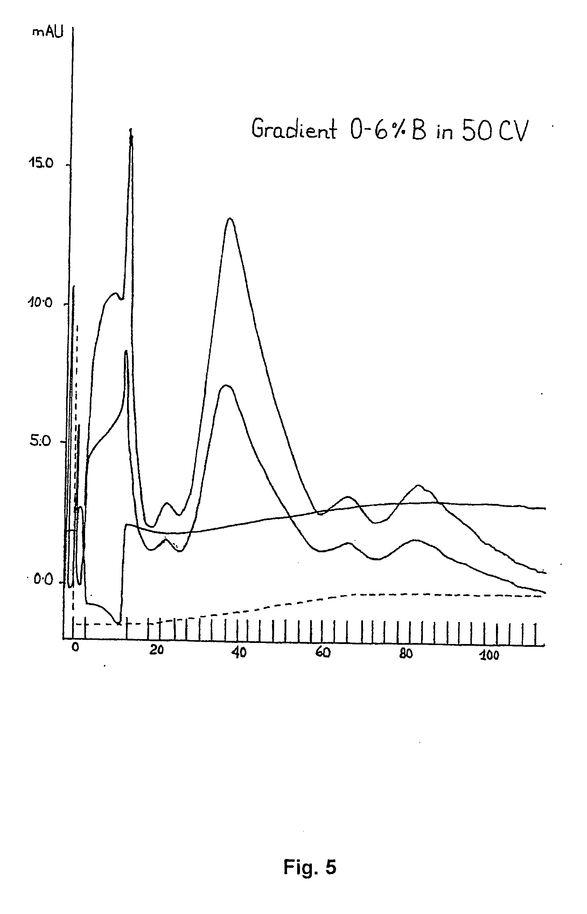

The different bio-physical properties between Met-IL21, IL21 and pyro-glutamine IL21 can be used for purification purposes/separation. Pyro-glutamine IL21 will due to the cyclized amid formation lack the normal protonation of the N-terminus. The (-1) charge difference between hIl-21 starting with pyro-glutamine and Met-IL21 can be used on a cation exchange column that will elute pyro-glutamine IL21 first (due to its lack of one positive charge) and subsequently Met-IL21 which displays a stronger binding to the cation resin. Further, in non-cyclized IL21 the N-terminally positioned glutamine will have the ability to form a hydrogen bond between the side chain amide oxygen and the charged N-terminal backbone amine, and thereby masking the charge at the N-terminus. Met-IL21 will not poses the ability for a similar charge masking and will therefore bind stronger to a cation exchange column than IL21. A mixture of Met-IL21, IL21 and pyro-glytamine IL21 including 300 mM NaCl and buffered at pH 6.5 was loaded on a Mono-S column. The A buffer consisted of 300mM NaCl buffered at pH 6.5 and the B buffer 1 M NaCl buffered at pH 6.5. A linear gradient (performed on an AKTA system) from 0-20% B buffer was applied over 45 column volumes. The fractions was assayed as described under the Q-cyclase example above. Using the above described gradient, efficient separation of Met-IL21, IL21. pyro-glutamine IL21 was achieved.(Figure 5)

### Met-hGH

Purified Met-AP (M206A) is used to remove the initiator Methionine from partly or fully purified Met-hGH (human growth hormone) where the P1' is a Phe residue. The cleavage is performed in a reaction buffer typically consisting of the following components: 2-100 mM K₂SO₄, 2-500 mM NaCl, 1-100 µM ZnCl₂ and 2-30 mM Hepes buffer pH 6-8. The cleavage is assayed by MALDI-TOF spectroscopy. The time of reaction is 2-66 hours. Using these conditions partly or full removal of Methionine from Met-hGH is demonstrated.

### Met-hGH

Purified Met-AP (M206A, Q233A) is used to remove the initiator

Methionine from partly or fully purified Met-hGH (human growth hormone) where the P1' is a Phe residue. The cleavage is performed in a reaction buffer typically consisting of the following components: 2-100 mM K₂SO₄, 2-500 mM NaCl, 1-100 µM ZnCl₂ and 2-30 mM Hepes buffer pH 6-8. The cleavage is assayed by MALDI-TOF spectroscopy. The time of reaction is 2-66 hours. Using these conditions partly or full removal of Methionine from Met-hGH is achieved.

### Met-IL-20

Purified Met-AP (M206A) is used to remove the initiator Methionine from partly or fully purified Met-IL-20 where the P1' is a Leu residue. The cleavage is performed in a reaction buffer typically consisting of the following components: 2-100 mM K₂SO₄, 2-500 mM NaCl, 1-100 µM ZnCl₂ and 2-30 mM Hepes buffer pH 6-8. The cleavage is assayed by MALDI-TOF spectroscopy. The time of reaction is 2-66 hours. Using these conditions partly or full removal of Methionine from Met-IL-20 is demonstrated.

### Met-IL-20

Purified Met-AP (M206A, Q233A) is used to remove the initiator Methionine from partly or fully purified Met-IL-20 where the P1' is a Leu residue. The cleavage is performed in a reaction buffer typically consisting of the following components: 2-100 mM K₂SO₄, 2-500 µM NaCl, 1-100 µM ZnCl₂ and 2-30 mM Hepes buffer pH 6-8. The cleavage is assayed by MALDI-TOF spectroscopy. The time of reaction is 2-66 hours. Using these conditions partly or full removal of Methionine from Met-IL-20 is demonstrated.

The excess subject-matter not covered by the claims is not part of the matter for which protection is sought but is left only for illustrative purposes.

## Claims

1. A method for removal of initiator methionine from a substrate protein and subsequent purification of the product wherein the removal of the initiator methionine is performed by use of an E. coli methionine aminopeptidase, wherein at least one of the residues Y168, M206 and Q233 has been amended into a sequence different from Y168,and M206 and Q233, and wherein the method for separating the substrate protein starting with Met-Gln from the product protein, identical to the substrate but starting with Gln, comprises the step of applying Qcyclase to convert the N-terminal Gln of the product into pyroglutamine, followed by purification utilising the charge difference between the substrate and the product.

2. A method according to claim 1, wherein the E. coli methionine aminopeptidase comprises an amendment of the amino acid in position 168.

3. A method according to claim 1, wherein the E. coli methionine aminopeptidase comprises an amendment in position 206.

4. A method according to claim 1, wherein the E. coli methionine aminopeptidase comprises an amendment in position 233.

5. A method according to claim 1, claim 3, or claim 4, wherein the E. coli methionine aminopeptidase comprises amendments in position 206 and 233.

6. A method according to any of claims 1 to 3, wherein the E. coli methionine aminopeptidase comprises amendments in positions 168 and 206.

7. A method according to any one of claims 1 to 2 or claim 4, wherein the E. coli methionine aminopeptidase comprises amendments in positions 168 and 233.

8. A method according to any one of claims 1 to 4, wherein the E. coli methionine aminopeptidase comprises amendments in positions 168, 206, and 233.

9. A method according to any of claims 1 to 8, wherein the E. coli methionine aminopeptidase comprises amendments comprises exchange of wildtype amino acid into Gly, Ala, Ser, Thr, Asn, or Asp.

10. A method according to any of claims 1 to 9, wherein the E. coli methionine aminopeptidase comprises exchange of wildtype amino acid into Ala and/or Gly.

11. A method according to claim 10, wherein the E. coli methionine aminopeptidase comprises exchanges of wildtype amino acid into Ala.

12. A method according to any of claims 1 to 11, wherein position 168 of the E. coli methionine aminopeptidase is Ala.

13. A method according to any of claims 1 to 11, wherein position 206 of the E. coli methionine aminopeptidase is Ala.

14. A method according to any of claims 1 to 11, wherein position 233 of the E. coli methionine aminopeptidase is Ala.

15. A method according to any of claims 1 to 11, wherein positions 206 and 233 of the E. coli methionine aminopeptidase are both Ala.

16. A method according to claim 1, wherein the E. coli methionine aminopeptidase has the amino acid sequence shown in Seq. id. No. 3.

17. A method according to claim 1, wherein the E. coli methionine aminopeptidase has the amino acid sequence shown in Seq. id. No. 5.

18. A method according to claim 1, wherein the E. coli methionine aminopeptidase has the amino acid sequence shown in Seq. id. No. 7

19. A method according to claim 1, wherein the E. coli methionine aminopeptidase has the amino acid sequence shown in Seq. id. No 9.

20. A method according to any of claims 1 to 19 comprising purification on a cation exchange column.

## Patentansprüche

1. Verfahren zum Entfernen von Initiator-Methionin von einem Substratprotein und anschließender Reinigung des Produkts, wobei die Entfernung des Initiator-Methionins durch Verwendung einer Methioninaminopeptidase von E. coli durchgeführt wird, wobei mindestens einer der Reste Y168 und M206 und Q233 zu einer anderen Sequenz als Y168, M206 und Q233 abgeändert worden ist und wobei das Verfahren zum Abtrennen des Substratproteins, ausgehend mit Met-Gln vom Protuktprotein, identisch mit dem Substrat, aber ausgehend mit Gln, den Schritt des Anwendens von Q-Cyclase zum Umwandeln des N-terminalen Gln des Produkts zu Pyroglyutamin, gefolgt von der Reinigung unter Ausnutzung des Ladungsunterschieds zwischen dem Substrat und dem Produkt umfasst.

2. Verfahren nach Anspruch 1, wobei die Methioninaminopeptidase von E. coli eine Abänderung der Aminosäure in Position 168 umfasst.

3. Verfahren nach Anspruch 1, wobei die Methioninaminopeptidase von E. coli eine Abänderung in Position 206 umfasst.

4. Verfahren nach Anspruch 1, wobei die Methioninaminopeptidase von E. coli eine Abänderung in Position 233 umfasst.

5. Verfahren nach Anspruch 1, Anspruch 3 oder Anspruch 4, wobei die Methioninaminopeptidase von E. coli Abänderungen in den Positionen 206 und 233 umfasst.

6. Verfahren nach einem der Ansprüche 1 bis 3, wobei die Methioninaminopeptidase von E. coli Abänderungen in den Positionen 168 und 206 umfasst.

7. Verfahren nach einem der Ansprüche 1 bis 2 oder Anspruch 4, wobei die Methioninaminopeptidase von E. coli Abänderungen in den Positionen 168 und 233 umfasst.

8. Verfahren nach einem der Ansprüche 1 bis 4, wobei die Methioninaminopeptidase von E. coli Abänderungen in den Positionen 168, 206 und 233 umfasst.

9. Verfahren nach einem der Ansprüche 1 bis 8, wobei die Methioninaminopeptidase von E. coli Abänderungen aufweist, die den Austausch der Wildtyp-Aminosäure zu Gly, Ala, Ser, Thr, Asn oder Asp umfasst.

10. Verfahren nach einem der Ansprüche 1 bis 9, wobei die Methioninaminopeptidase von E. coli den Austausch der Wildtyp-Aminosäure zu Ala und/oder Gly umfasst.

11. Verfahren nach Anspruch 10, wobei die Methioninaminopeptidase von E. coli den Austausch der Wildtyp-Aminosäure zu Ala umfasst.

12. Verfahren nach einem der Ansprüche 1 bis 11, wobei Position 168 der Methioninaminopeptidase von E. coli Ala ist.

13. Verfahren nach einem der Ansprüche 1 bis 11, wobei Position 206 der Methioninaminopeptidase von E. coli Ala ist.

14. Verfahren nach einem der Ansprüche 1 bis 11, wobei Position 233 der Methioninaminopeptidase von E. coli Ala ist.

15. Verfahren nach einem der Ansprüche 1 bis 11, wobei beide Positionen 206 und 233 der Methioninaminopeptidase von E. coli Ala sind.

16. Verfahren nach Anspruch 1, wobei die Methioninaminopeptidase von E. coli die in SEQ. ID. No. 3 dargestellte Aminosäuresequenz aufweist.

17. Verfahren nach Anspruch 1, wobei die Methioninaminopeptidase von E. coli die in SEQ. ID. No. 5 dargestellte Aminosäuresequenz aufweist.

18. Verfahren nach Anspruch 1, wobei die Methioninaminopeptidase von E. coli die in SEQ. ID. No. 7 dargestellte Aminosäuresequenz aufweist.

19. Verfahren nach Anspruch 1, wobei die Methioninaminopeptidase von E. coli die in SEQ. ID. No. 9 dargestellte Aminosäuresequenz aufweist.

20. Verfahren nach einem der Ansprüche 1 bis 19, umfassend die Reinigung auf einer Kationenaustauschsäule.

## Revendications

1. Procédé pour l'élimination d'une protéine formant substrat un initiateur méthionine, et la purification ultérieure du produit, l'élimination de l'initiateur méthionine étant réalisée par utilisation d'une méthionine aminopeptidase de E. coli, où au moins l'un des résidus Y168, M206 et Q233 a été modifié pour donner une séquence différente de Y168, et M206 et Q233, et où le procédé de séparation de la protéine formant substrat, en partant de Met-Gln provenant de la protéine produit, qui est identique au substrat mais qui commence par Gln, comprend l'étape consistant à appliquer de la Qcyclase dans le but de convertir le Gln N-terminal du produit en pyroglutamine, puis de purification par utilisation de la différence de charge entre le substrat et le produit.

2. Procédé selon la revendication 1, dans lequel la méthionine aminopeptidase de E. coli comprend une modification de l'acide aminé en position 168.

3. Procédé selon la revendication 1, dans lequel la méthionine aminopeptidase de E. coli comprend une modification en position 206.

4. Procédé selon la revendication 1, dans lequel la méthionine aminopeptidase de E. coli comprend une modification en position 233.

5. Procédé selon la revendication 1, 3 ou 4, dans lequel la méthionine aminopeptidase de E. coli comprend des modifications en positions 206 et 233.

6. Procédé selon l'une quelconque des revendications 1 à 3, dans lequel la méthionine aminopeptidase de E. coli comprend des modifications en positions 168 et 206.

7. Procédé selon l'une quelconque des revendications 1 à 2 ou 4, dans lequel la méthionine aminopeptidase de E. coli comprend des modifications en positions 168 et 233.

8. Procédé selon l'une quelconque des revendications 1 à 4, dans lequel la méthionine aminopeptidase de E. coli comprend des modifications en positions 168, 206 et 233.

9. Procédé selon l'une quelconque des revendications 1 à 8, dans lequel la méthionine aminopeptidase de E. coli comprend des modifications qui comprennent un remplacement de l'acide aminé de type sauvage par Gly, Ala, Ser, Thr, Asn ou Asp.

10. Procédé selon l'une quelconque des revendications 1 à 9, dans lequel la méthionine aminopeptidase de E. coli comprend un remplacement d'un acide aminé de type sauvage par Ala et/ou Gly.

11. Procédé selon la revendication 10, dans lequel la méthionine aminopeptidase de E. coli comprend des remplacements d'un acide aminé de type sauvage par Ala.

12. Procédé selon l'une quelconque des revendications 1 à 11, dans lequel la position 168 de la méthionine aminopeptidase de E. coli est Ala.

13. Procédé selon l'une quelconque des revendications 1 à 11, dans lequel la position 206 de la méthionine aminopeptidase de E. coli est Ala.

14. Procédé selon l'une quelconque des revendications 1 à 11, dans lequel la position 233 de la méthionine aminopeptidase de E. coli est Ala.

15. Procédé selon l'une quelconque des revendications 1 à 11, dans lequel les positions 206 et 233 de la méthionine aminopeptidase de E. coli sont toutes les deux Ala.

16. Procédé selon la revendication 1, dans lequel la méthionine aminopeptidase de E. coli a la séquence d'acides aminés présentée en SEQ ID N°3.

17. Procédé selon la revendication 1, dans lequel la méthionine aminopeptidase de E. coli a la séquence d'acides aminés présentée en SEQ ID N°5.

18. Procédé selon la revendication 1, dans lequel la méthionine aminopeptidase de E. coli a la séquence d'acides aminés présentée en SEQ ID N°7.

19. Procédé selon la revendication 1, dans lequel la méthionine aminopeptidase de E. coli a la séquence d'acides aminés présentée en SEQ ID N°9.

20. Procédé selon l'une des revendications 1 à 19, comprenant une purification sur une colonne d'échange de cations.


### SEQUENCE LISTING

<110> Novo Nordisk A/S
<120> Processing of peptides
<130> 6744
<140> PA 2003 01892
   <141> 2003-12-19
<150> PA 2003 01892
   <151> 2003-12-19
<160> 9
<170> Patent In version 3.2
<210> 1
   <211> 264
   <212> PRT
   <213> Escherichia coli
<220>
   <221> misc_feature
   <222> (168)..(168)
   <223> Xaa can be any naturally occurring amino acid
<220>
   <221> misc_feature
   <222> (206)..(206)
   <223> aa can be any naturally occurring amino acid
<220>
   <221> misc_feature
   <222> (233)..(233)
   <223> Xaa can be any naturally occurring amino acid
<400> 1
   Met Ala Ile Ser Ile Lys Thr Pro Glu Asp Ile Glu Lys Met Arg Val
   1               5                   10                  15
   Ala Gly Arg Leu Ala Ala Glu Val Leu Glu Met Ile Glu Pro Tyr Val
               20                  25                  30
   Lys Pro Gly Val Ser Thr Gly Glu Leu Asp Arg Ile Cys Asn Asp Tyr
           35                  40                  45
   Ile Val Asn Glu Gln His Ala Val Ser Ala Cys Leu Gly Tyr His Gly
       50                  55                  60
   Tyr Pro Lys Ser Val Cys Ile Ser Ile Asn Glu Val Val Cys His Gly
   65                  70                  75                  80
   Ile Pro Asp Asp Ala Lys Leu Leu Lys Asp Gly Asp Ile Val Asn Ile
                   85                  90                  95
   Asp Val Thr Val Ile Lys Asp Gly Phe His Gly Asp Thr Ser Lys Met
               100                 105                 110
   Phe Ile Val Gly Lys Pro Thr Ile Met Gly Glu Arg Leu Cys Arg Ile
           115                 120                 125
   Thr Gln Glu Ser Leu Tyr Leu Ala Leu Arg Met Val Lys Pro Gly Ile
       130                 135                 140
   Asn Leu Arg Glu Ile Gly Ala Ala Ile Gln Lys Phe Val Glu Ala Glu
   145                 150                 155                 160
   Gly Phe Ser Val Val Arg Glu Xaa Cys Gly His Gly Ile Gly Arg Gly
                   165                 170                 175
   Phe His Glu Glu Pro Gln Val Leu His Tyr Asp Ser Arg Glu Thr Asn
               180                 185                 190
   Val Val Leu Lys Pro Gly Met Thr Phe Thr Ile Glu Pro Xaa Val Asn
           195                 200                 205
   Ala Gly Lys Lys Glu Ile Arg Thr Met Lys Asp Gly Trp Thr Val Lys
       210                 215                 220
   Thr Lys Asp Arg Ser Leu Ser Ala Xaa Tyr Glu His Thr Ile Val Val
   225                 230                 235                 240
   Thr Asp Asn Gly Cys Glu Ile Leu Thr Leu Arg Lys Asp Asp Thr Ile
                   245                 250                 255
   Pro Ala Ile Ile Ser His Asp Glu
               260
<210> 2
   <211> 792
   <212> DNA
   <213> Escherichia coli
<400> 2
   atggctatct caatcaagac cccagaagat atcgaaaaaa tgcgcgtcgc tggccgactg 60
   gctgccgaag tgctggagat gatcgaaccg tatgttaaac cgggcgtcag caccggcgag 120
   ctggatcgca tctgtaatga ttacattgtt aatgaacaac acgcggtttc tgcctgcctc 180
   ggctatcacg gctatccgaa atccgtttgc atctctatta atgaagtggt gtgccacggt 240
   atcccggacg atgctaagct gctgaaagat ggcgatatcg ttaacattga tgtcaccgta 300
   atcaaagatg gtttccacgg cgatacctcg aaaatgttta tcgtcggtaa gccgaccatc 360
   atgggcgaac gtctgtgccg catcacgcaa gaaagcctgt acctggcgct acgcatggta 420
   aaaccaggca ttaatctgcg cgaaatcggt gcggcgattc agaaatttgt cgaagcagaa 480
   ggcttctccg tcgttcgtga atattgcgga cacggtattg gtcgcggctt ccatgaagaa 540
   ccgcaggtgc tgcactatga ctcccgtgaa accaacgtcg tactgaaacc tgggatgacg 600
   ttcaccatcg agccagcggt caacgcgggt aaaaaagaga tccgcaccat gaaagatggc 660
   tggacggtaa aaaccaaaga tcgcagcttg tctgcacagt atgagcatac tattgtggtg 720
   actgataacg gctgcgaaat tctgacgcta cgcaaggatg acaccatccc ggcgataatc 780
   tcgcacgacg aa 792
<210> 3
   <211> 264
   <212> PRT
   <213> Escherichia coli
<400> 3
   Met Ala Ile Ser Ile Lys Thr Pro Glu Asp Ile Glu Lys Met Arg Val
   1               5                   10                  15
   Ala Gly Arg Leu Ala Ala Glu Val Leu Glu Met Ile Glu Pro Tyr Val
               20                  25                  30
   Lys Pro Gly Val Ser Thr Gly Glu Leu Asp Arg Ile Cys Asn Asp Tyr
           35                  40                  45
   Ile Val Asn Glu Gln His Ala Val Ser Ala Cys Leu Gly Tyr His Gly
       50                  55                  60
   Tyr Pro Lys Ser Val Cys Ile Ser Ile Asn Glu Val Val Cys His Gly
   65                  70                  75                  80
   Ile Pro Asp Asp Ala Lys Leu Leu Lys Asp Gly Asp Ile Val Asn Ile
                   85                  90                  95
   Asp Val Thr Val Ile Lys Asp Gly Phe His Gly Asp Thr Ser Lys Met
               100                 105                 110
   Phe Ile Val Gly Lys Pro Thr Ile Met Gly Glu Arg Leu Cys Arg Ile
           115                 120                 125
   Thr Gln Glu Ser Leu Tyr Leu Ala Leu Arg Met Val Lys Pro Gly Ile
       130                 135                 140
   Asn Leu Arg Glu Ile Gly Ala Ala Ile Gln Lys Phe Val Glu Ala Glu
   145                 150                 155                 160
   Gly Phe Ser Val Val Arg Glu Tyr Cys Gly His Gly Ile Gly Arg Gly
                   165                 170                 175
   Phe His Glu Glu Pro Gln Val Leu His Tyr Asp Ser Arg Glu Thr Asn
               180                 185                 190
   Val Val Leu Lys Pro Gly Met Thr Phe Thr Ile Glu Pro Ala Val Asn
           195                 200                 205
   Ala Gly Lys Lys Glu Ile Arg Thr Met Lys Asp Gly Trp Thr Val Lys
   210                                    215                                    220
   Thr Lys Asp Arg Ser Leu Ser Ala Gln Tyr Glu His Thr Ile Val Val
   225                 230                 235                 240
   Thr Asp Asn Gly Cys Glu Ile Leu Thr Leu Arg Lys Asp Asp Thr Ile
                   245                 250                 255
   Pro Ala Ile Ile Ser His Asp Glu
               260
<210> 4
   <211> 792
   <212> DNA
   <213> Escherichia coli
<400> 4
   atggctatct caatcaagac cccagaagat atcgaaaaaa tgcgcgtcgc tggccgactg     60
   gctgccgaag tgctggagat gatcgaaccg tatgttaaac cgggcgtcag caccggcgag    120
   ctggatcgca tctgtaatga ttacattgtt aatgaacaac acgcggtttc tgcctgcctc    180
   ggctatcacg gctatccgaa atccgtttgc atctctatta atgaagtggt gtgccacggt    240
   atcccggacg atgctaagct gctgaaagat ggcgatatcg ttaacattga tgtcaccgta    300
   atcaaagatg gtttccacgg cgatacctcg aaaatgttta tcgtcggtaa gccgaccatc    360
   atgggcgaac gtctgtgccg catcacgcaa gaaagcctgt acctggcgct acgcatggta    420
   aaaccaggca ttaatctgcg cgaaatcggt gcggcgattc agaaatttgt cgaagcagaa    480
   ggcttctccg tcgttcgtga atattgcgga cacggtattg gtcgcggctt ccatgaagaa    540
   ccgcaggtgc tgcactatga ctcccgtgaa accaacgtcg tactgaaacc tgggatgacg    600
   ttcaccatcg agccaatggt caacgcgggt aaaaaagaga tccgcaccat gaaagatggc    660
   tggacggtaa aaaccaaaga tcgcagcttg tctgcagcgt atgagcatac tattgtggtg    720
   actgataacg gctgcgaaat tctgacgcta cgcaaggatg acaccatccc ggcgataatc    780
   tcgcacgacg aa                                                        792
<210> 5
   <211> 264
   <212> PRT
   <213> Escherichia coli
<400> 5
   Met Ala Ile Ser Ile Lys Thr Pro Glu Asp Ile Glu Lys Met Arg Val
   1               5                   10                  15
   Ala Gly Arg Leu Ala Ala Glu Val Leu Glu Met Ile Glu Pro Tyr Val
               20                  25                  30
   Lys Pro Gly Val Ser Thr Gly Glu Leu Asp Arg Ile Cys Asn Asp Tyr
           35                  40                  45
   Ile Val Asn Glu Gln His Ala Val Ser Ala Cys Leu Gly Tyr His Gly
   50 55 60
   Tyr Pro Lys Ser Val Cys Ile Ser Ile Asn Glu Val Val Cys His Gly
   65 70 75 80
   Ile Pro Asp Asp Ala Lys Leu Leu Lys Asp Gly Asp Ile Val Asn Ile
   85 90 95
   Asp Val Thr Val Ile Lys Asp Gly Phe His Gly Asp Thr Ser Lys Met
   100 105 110
   Phe Ile Val Gly Lys Pro Thr Ile Met Gly Glu Arg Leu Cys Arg Ile
   115 120 125
   Thr Gln Glu Ser Leu Tyr Leu Ala Leu Arg Met Val Lys Pro Gly Ile
   130 135 140
   Asn Leu Arg Glu Ile Gly Ala Ala Ile Gln Lys Phe Val Glu Ala Glu
   145 150 155 160
   Gly Phe Ser Val Val Arg Glu Tyr Cys Gly His Gly Ile Gly Arg Gly
   165 170 175
   Phe His Glu Glu Pro Gln Val Leu His Tyr Asp Ser Arg Glu Thr Asn
   180 185 190
   Val Val Leu Lys Pro Gly Met Thr Phe Thr Ile Glu Pro Met Val Asn
   195 200 205
   Ala Gly Lys Lys Glu Ile Arg Thr Met Lys Asp Gly Trp Thr Val Lys
   210 215 220
   Thr Lys Asp Arg Ser Leu Ser Ala Ala Tyr Glu His Thr Ile Val Val
   225 230 235 240
   Thr Asp Asn Gly Cys Glu Ile Leu Thr Leu Arg Lys Asp Asp Thr Ile
   245 250 255
   Pro Ala Ile Ile Ser His Asp Glu
   260
<210> 6
   <211> 792
   <212> DNA
   <213> Escherichia coli
<400> 6
   atggctatct caatcaagac cccagaagat atcgaaaaaa tgcgcgtcgc tggccgactg      60
   gctgccgaag tgctggagat gatcgaaccg tatgttaaac cgggcgtcag caccggcgag     120
   ctggatcgca tctgtaatga ttacattgtt aatgaacaac acgcggtttc tgcctgcctc     180
   ggctatcacg gctatccgaa atccgtttgc atctctatta atgaagtggt gtgccacggt     240
   atcccggacg atgctaagct gctgaaagat ggcgatatcg ttaacattga tgtcaccgta     300
   atcaaagatg gtttccacgg cgatacctcg aaaatgttta tcgtcggtaa gccgaccatc     360
   atgggcgaac gtctgtgccg catcacgcaa gaaagcctgt acctggcgct acgcatggta     420
   aaaccaggca ttaatctgcg cgaaatcggt gcggcgattc agaaatttgt cgaagcagaa     480
   ggcttctccg tcgttcgtga atattgcgga cacggtattg gtcgcggctt ccatgaagaa     540
   ccgcaggtgc tgcactatga ctcccgtgaa accaacgtcg tactgaaacc tgggatgacg     600
   ttcaccatcg agccagcggt caacgcgggt aaaaaagaga tccgcaccat gaaagatggc     660
   tggacggtaa aaaccaaaga tcgcagcttg tctgcagcgt atgagcatac tattgtggtg     720
   actgataacg gctgcgaaat tctgacgcta cgcaaggatg acaccatccc ggcgataatc     780
   tcgcacgacg aa                                                         792
<210> 7
   <211> 264
   <212> PRT
   <213> Escherichia coli
<400> 7
   Met Ala Ile Ser Ile Lys Thr Pro Glu Asp Ile Glu Lys Met Arg Val
   1               5                   10                  15
   Ala Gly Arg Leu Ala Ala Glu Val Leu Glu Met Ile Glu Pro Tyr Val
               20                  25                  30
   Lys Pro Gly Val Ser Thr Gly Glu Leu Asp Arg Ile Cys Asn Asp Tyr
           35                  40                  45
   Ile Val Asn Glu Gln His Ala Val Ser Ala Cys Leu Gly Tyr His Gly
       50                  55                  60
   Tyr Pro Lys Ser Val Cys Ile Ser Ile Asn Glu Val Val Cys His Gly
   65                  70                  75                  80
   Ile Pro Asp Asp Ala Lys Leu Leu Lys Asp Gly Asp Ile Val Asn Ile
                   85                  90                  95
   Asp Val Thr Val Ile Lys Asp Gly Phe His Gly Asp Thr Ser Lys Met
               100                 105                 110
   Phe Ile Val Gly Lys Pro Thr Ile Met Gly Glu Arg Leu Cys Arg Ile
           115                 120                 125
   Thr Gln Glu Ser Leu Tyr Leu Ala Leu Arg Met Val Lys Pro Gly Ile
       130                 135                 140
   Asn Leu Arg Glu Ile Gly Ala Ala Ile Gln Lys Phe Val Glu Ala Glu
   145                 150                 155                 160
   Gly Phe Ser Val Val Arg Glu Tyr Cys Gly His Gly Ile Gly Arg Gly
                   165                 170                 175
   Phe His Glu Glu Pro Gln Val Leu His Tyr Asp Ser Arg Glu Thr Asn
               180                 185                 190
   Val Val Leu Lys Pro Gly Met Thr Phe Thr Ile Glu Pro Ala Val Asn
           195                 200                 205
   Ala Gly Lys Lys Glu Ile Arg Thr Met Lys Asp Gly Trp Thr Val Lys
       210                 215                 220
   Thr Lys Asp Arg Ser Leu Ser Ala Ala Tyr Glu His Thr Ile Val Val
   225                 230                 235                 240
   Thr Asp Asn Gly Cys Glu Ile Leu Thr Leu Arg Lys Asp Asp Thr Ile
                   245                 250                 255
   Pro Ala Ile Ile Ser His Asp Glu
               260
<210> 8
   <211> 795
   <212> DNA
   <213> Escherichia coli
<400> 8
   atggctatct caatcaagac cccagaagat atcgaaaaaa tgcgcgtcgc tggccgactg 60
   gctgccgaag tgctggagat gatcgaaccg tatgttaaac cgggcgtcag caccggcgag 120
   ctggatcgca tctgtaatga ttacattgtt aatgaacaac acgcggtttc tgcctgcctc 180
   ggctatcacg gctatccgaa atccgtttgc atctctatta atgaagtggt gtgccacggt 240
   atcccggacg atgctaagct gctgaaagat ggcgatatcg ttaacattga tgtcaccgta 300
   atcaaagatg gtttccacgg cgatacctcg aaaatgttta tcgtcggtaa gccgaccatc 360
   atgggcgaac gtctgtgccg catcacgcaa gaaagcctgt acctggcgct acgcatggta 420
   aaaccaggca ttaatctgcg cgaaatcggt gcggcgattc agaaatttgt cgaagcagaa 480
   ggcttctccg tcgttcgtga agcgtgcgga cacggtattg gtcgcggctt ccatgaagaa 540
   ccgcaggtgc tgcactatga ctcccgtgaa accaacgtcg tactgaaacc tgggatgacg 600
   ttcaccatcg agccaatggt caacgcgggt aaaaaagaga tccgcaccat gaaagatggc 660
   tggacggtaa aaaccaaaga tcgcagcttg tctgcacaat atgagcatac tattgtggtg 720
   actgataacg gctgcgaaat tctgacgcta cgcaaggatg acaccatccc ggcgataatc 780
   tcgcacgacg aataa 795
<210> 9
   <211> 264
   <212> PRT
   <213> Escherichia coli
<400> 9
   Met Ala Ile Ser Ile Lys Thr Pro Glu Asp Ile Glu Lys Met Arg Val
   1               5                   10                  15
   Ala Gly Arg Leu Ala Ala Glu Val Leu Glu Met Ile Glu Pro Tyr Val
               20                  25                  30
   Lys Pro Gly Val Ser Thr Gly Glu Leu Asp Arg Ile Cys Asn Asp Tyr
           35                  40                  45
   Ile Val Asn Glu Gln His Ala Val Ser Ala Cys Leu Gly Tyr His Gly
       50                  55                  60
   Tyr Pro Lys Ser Val Cys Ile Ser Ile Asn Glu Val Val Cys His Gly
   65                  70                  75                  80
   Ile Pro Asp Asp Ala Lys Leu Leu Lys Asp Gly Asp Ile Val Asn Ile
                   85                  90                  95
   Asp Val Thr Val Ile Lys Asp Gly Phe His Gly Asp Thr Ser Lys Met
               100                 105                 110
   Phe Ile Val Gly Lys Pro Thr Ile Met Gly Glu Arg Leu Cys Arg Ile
           115                 120                 125
   Thr Gln Glu Ser Leu Tyr Leu Ala Leu Arg Met Val Lys Pro Gly Ile
       130                 135                 140
   Asn Leu Arg Glu Ile Gly Ala Ala Ile Gln Lys Phe Val Glu Ala Glu
   145                 150                 155                 160
   Gly Phe Ser Val Val Arg Glu Ala Cys Gly His Gly Ile Gly Arg Gly
                   165                 170                 175
   Phe His Glu Glu Pro Gln Val Leu His Tyr Asp Ser Arg Glu Thr Asn
               180                 185                 190
   Val Val Leu Lys Pro Gly Met Thr Phe Thr Ile Glu Pro Met Val Asn
           195                 200                 205
   Ala Gly Lys Lys Glu Ile Arg Thr Met Lys Asp Gly Trp Thr Val Lys
       210                 215                 220
   Thr Lys Asp Arg Ser Leu Ser Ala Gln Tyr Glu His Thr Ile Val Val
   225                 230                 235                 240
   Thr Asp Asn Gly Cys Glu Ile Leu Thr Leu Arg Lys Asp Asp Thr Ile
                   245                 250                 255
   Pro Ala Ile Ile Ser His Asp Glu
                   260